# EUROPEAN PATENT APPLICATION

(11) **EP 4 353 327 A1**
(43) Date of publication of application: **17.04.2024**
(21) Application number: 22811170.4
(22) Date of filing: 13.05.2022
(51) Int. Cl.: A61Q 13/00, A61Q 19/10, C11D 3/50, A61Q 5/02, A61K 8/34, A61K 8/49, D06M 13/00, C11B 9/00

(54) **PERFUME COMPOSITION**

(30) Priority: 24.05.2021 JP 2021086906
(71) Applicant: Kao Corporation, Chuo-ku Tokyo 103-8210 (JP)
(72) Inventor: SAITO Akari, Tokyo 131-8501 (JP); MORI Hironori, Tokyo 131-8501 (JP); DEGUCHI Jun, Tokyo 131-8501 (JP); ARAI Tsubasa, Wakayama 6408580 (JP)
(74) Representative: Hoffmann Eitle
(86) International application number: PCT/JP2022/020175
(87) International publication number: WO 2022/249897

(57) **Abstract**

Provided are a perfuming agent composed of a compound represented by Formula (I), and a fragrance composition containing the perfuming agent. 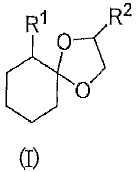 (In Formula (I), R¹ is an alkyl group having 1 or more and 5 or less of carbon atoms, and R² is a hydrogen atom or an alkyl group having 1 or more and 4 or less of carbon atoms.)

## Description

### Technical Field

The present invention relates to a fragrance composition.

### Background Art

α-(2-Alkylcyclohexyloxy)-β-alkanols, of which especially 1-(2-t-butylcyclohexyloxy)-2-alkanol, are useful fragrance materials that have a woody, amber-like aromatic odor, exhibit excellent persistence of odor, and can be produced at low cost (JP 2013-151482A).

For example, 1-(2-t-butylcyclohexyloxy)-2-butanol is widely known as Amber Core, a fragrance that has woody and amber-like notes (Japanese cypress-like fresh odor) as well as long-term persistence of odor.

In production of Amber Core, various by-products are produced, although the amounts thereof are very small. Some of such by-products are very similar to Amber Core in their structures, and thus it is difficult to remove them by purification. Moreover, some of such by-products are known to adversely affect the aromatic odor of Amber Core.

### Disclosure of Invention

The present invention relates to a perfuming agent composed of a compound represented by Formula (I).

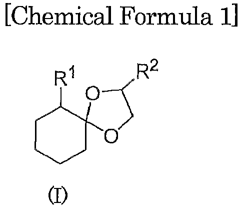

In Formula (I),
R¹ is an alkyl group having 1 or more and 5 or less of carbon atoms, and
R² is a hydrogen atom or an alkyl group having 1 or more and 4 or less of carbon atoms.

### Description of the Invention

It is an object of the present invention to provide a novel perfuming agent and a novel fragrance composition by studying conventional techniques for producing Amber Core.

The inventors of the present invention have found that o-t-butyl cyclohexanone generated during the production of Amber Core and having a low boiling point is a component with an unpleasant odor. On the other hand, they have also newly found that certain types of ketal products have favorable fruity (apple-like), herbal, and minty odors by themselves. Further, they have found that, when such a ketal product is contained at a specific concentration in a composition containing Amber Core, it brings about an advantageous effect in that nectar-like sweetness and voluminousness of the odor are imparted to the Amber Core. Based on these findings, the inventors finally devised a perfuming agent and a fragrance composition according to the present invention.

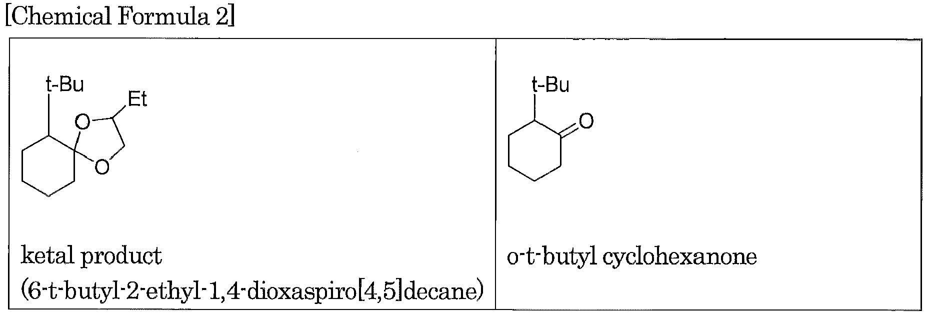

The following compounds are known as similar compounds having ketal structures. The odors of these compounds have been described as follows.

JP S56(1981)-79686A discloses a ketal compound shown below. It has been reported that this compound has highly pungent, medicinal herb-like and fruity odors. However, there is a problem in that impressions of medicinal herb-like highly pungent and chemical odors are not widely usable in preparation of fragrance blends.

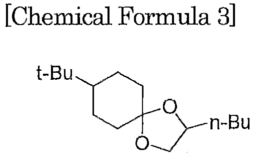

JP H6(1994)-305999A discloses a ketal compound shown below. It has been reported that this compound has fruity, green, woody, floral, anise-like, aromatic, camphor-like, and tobacco-like odors. However, there is a problem in that impressions of anise-like, camphor-like, and tobacco-like odors are not widely usable in preparation of fragrance blends.

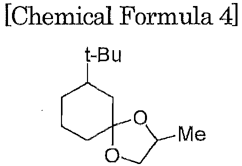

That is, the present invention relates to a perfuming agent composed of a compound represented by Formula (I).

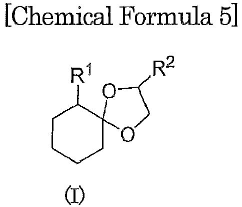

In Formula (I),
R¹ is an alkyl group having 1 or more and 5 or less of carbon atoms, and
R² is a hydrogen atom or an alkyl group having 1 or more and 4 or less of carbon atoms.

The present invention also relates to a fragrance composition containing: a compound represented by Formula (II) and a compound represented by Formula (I), wherein a mass ratio between the compound represented by Formula (II) and the compound represented by Formula (I) (the compound represented by Formula (II) : the compound represented by Formula (I)) is 99.99:0.01 or more and 98:2 or less.

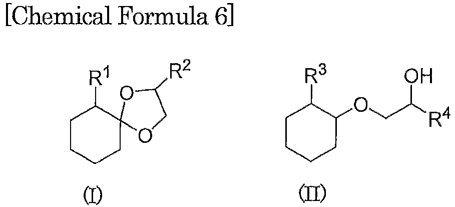

In Formulae (I) and (II),
R¹ and R³ are each independently an alkyl group having 1 or more and 5 or less of carbon atoms, and
R² and R⁴ are each independently a hydrogen atom or an alkyl group having 1 or more and 4 or less of carbon atoms.

The perfuming agent composed of the compound represented by Formula (I) and the fragrance composition containing the perfuming agent according to the present invention have a favorable fruity (apple-like), herbal, and minty odor. In addition, the fragrance composition containing the compound represented by Formula (II) and the compound represented by Formula (I) according to the present invention brings about an advantageous effect in that nectar-like sweetness and voluminousness of the odor are imparted to Amber Core.

As described above, the present invention relates to a perfuming agent composed of a compound represented by Formula (I).

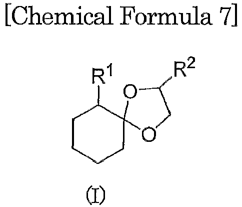

In Formula (I),
R¹ is an alkyl group having 1 or more and 5 or less of carbon atoms, and
R² is a hydrogen atom or an alkyl group having 1 or more and 4 or less of carbon atoms.

It is preferable that, in Formula (I),
R¹ is an alkyl group having 1 or more and 4 or less of carbon atoms, and
R² is a methyl group or an ethyl group.

It is more preferable that, in Formula (I),
R¹ is a tert-butyl group, and
R² is an ethyl group.

The compound represented by Formula (I) can be produced by, for example, a method to be described below.

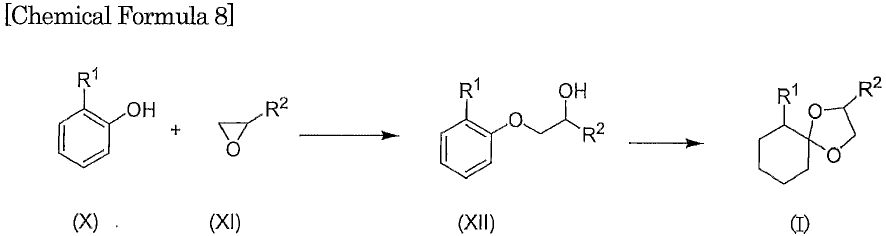

In Formulae (X), (XI), (XII), and (I),
R¹ is an alkyl group having 1 or more and 5 or less of carbon atoms, and
R² is a hydrogen atom or an alkyl group having 1 or more and 4 or less of carbon atoms.

A compound of Formula (X) and a compound of Formula (XI) are heated under basic conditions (e.g., in the presence of sodium hydroxide) to obtain a compound of Formula (XII). The compound of Formula (XII) is then hydrogenated in the presence of hydrogen using a specific catalyst (e.g., a palladium-containing catalyst), whereby a compound of Formula (I) can be obtained. The thus-obtained compound of Formula (I) can be separated and purified by distillation or column chromatography.

Alternatively, the compound represented by Formula (I) can be produced by a conventionally known method such as, for example, a method performed using the same conditions as those described in JP 2013-151482A.

The present invention also relates to a fragrance composition containing: a compound represented by Formula (II) and a compound represented by Formula (I), wherein a mass ratio between the compound represented by Formula (II) and the compound represented by Formula (I) (the compound represented by Formula (II) : the compound represented by Formula (I)) is 99.99:0.01 or more and 98:2 or less. That is, the mass ratio between the compound represented by Formula (I) and the compound represented by Formula (II) [the compound represented by Formula (I)/the compound represented by Formula (II)] is 0.01/99.99 or more and 2/98 or less.

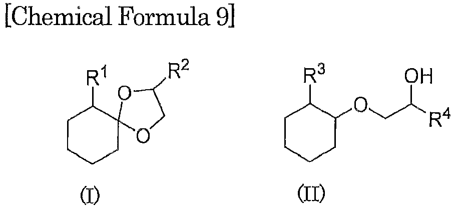

In Formulae (I) and (II),
R¹ and R³ are each independently an alkyl group having 1 or more and 5 or less of carbon atoms, and
R² and R⁴ are each independently a hydrogen atom or an alkyl group having 1 or more and 4 or less of carbon atoms.

In the fragrance composition containing the compound represented by Formula (II) and the compound represented by Formula (I), the mass ratio between the compound represented by Formula (II) and the compound represented by Formula (I) (the compound represented by Formula (II) : the compound represented by Formula (I)) is preferably 99.97:0.03 or more and more preferably 99.95:0.05 or more from the viewpoint of imparting nectar-like sweetness and voluminousness of the odor to the compound represented by Formula (II), and is preferably 99:1 or less, more preferably 99.20:0.80 or less, and still more preferably 99.50:0.50 or less from the viewpoint of imparting an amber-like odor of the compound represented by Formula (II). In the fragrance composition containing the compound represented by Formula (II) and the compound represented by Formula (I), the mass ratio between the compound represented by Formula (II) and the compound represented by Formula (I) (the compound represented by Formula (II) : the compound represented by Formula (I)) is preferably 99.99:0.01 or more and 99:1 or less, more preferably 99.97:0.03 or more and 99.20^{:}0.80 or less, and still more preferably 99.95:0.05 or more and 99.50:0.50 or less from the viewpoint of imparting nectar-like sweetness and voluminousness of the odor to the compound represented by Formula (II).

In other words, the mass ratio between the compound represented by Formula (I) and the compound represented by Formula (II) (the compound represented by Formula (I)/the compound represented by Formula (II)) is 0.01/99.99 or more and 1/99 or less, more preferably 0.03/99.97 or more and 0.80/99.2 or less, and still more preferably 0.05/99.95 or more and 0.50/99.50 or less from the viewpoint of imparting nectar-like sweetness and voluminousness of the odor to the compound represented by Formula (II).

In the fragrance composition containing the compound represented by Formula (II) and the compound represented by Formula (I), it is preferable that, in Formula (I),
R¹ is an alkyl group having 1 or more and 4 or less of carbon atoms, and
R² is a methyl group or an ethyl group.

It is more preferable that, in Formula (I),
R¹ is a tert-butyl group, and
R² is an ethyl group.

In the fragrance composition containing the compound represented by Formula (II) and the compound represented by Formula (I), it is preferable that, in Formula (II),
R³ is an alkyl group having 1 or more and 4 or less of carbon atoms, and
R⁴ is a methyl group or an ethyl group.

It is more preferable that, in Formula (II),
R³ is a tert-butyl group, and
R⁴ is an ethyl group.

In the fragrance composition containing the compound represented by Formula (II) and the compound represented by Formula (I), it is preferable that, in Formula (I),
R¹ is an alkyl group having 1 or more and 4 or less of carbon atoms, and
R² is a methyl group or an ethyl group, and
in Formula (II),
R³ is an alkyl group having 1 or more and 4 or less of carbon atoms, and
R⁴ is a methyl group or an ethyl group.

It is more preferable that, in Formula (I),
R¹ is a tert-butyl group, and
R² is an ethyl group, and
in Formula (II),
R³ is a tert-butyl group, and
R⁴ is an ethyl group.

The compound represented by Formula (II) can be produced by a conventionally known method such as, for example, the method described in JP H4(1992)-217937A.

Alternatively, the compound represented by Formula (II) can be produced by, for example, a method to be described below.

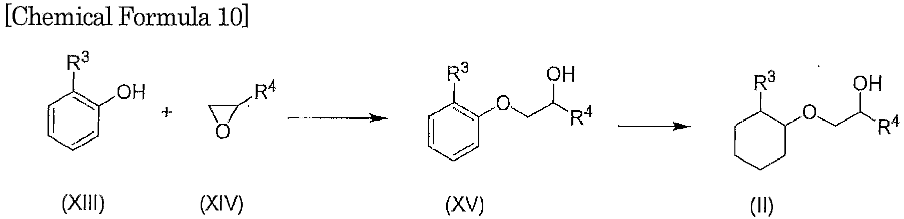

In Formulae (XIII), (XIV), (XV), and (II),
R³ is an alkyl group having 1 or more and 5 or less of carbon atoms, and
R⁴ is a hydrogen atom or an alkyl group having 1 or more and 4 or less of carbon atoms.

A compound of Formula (XIII) and a compound of Formula (XIV) are heated in the presence of a base (e.g., sodium hydroxide), whereby a compound of Formula (XV) can be obtained. The compound of Formula (XV) is then hydrogenated in the presence of a specific catalyst (e.g., a palladium catalyst) and hydrogen, whereby a compound of Formula (II) can be obtained.

The perfuming agent composed of the compound represented by Formula (I) according to the present invention has a favorable fruity (apple-like), herbal, and minty odor. Thus, it harmonizes well with various other fragrances and can bring about a characteristic effect on odors when used in fragrance blending. The perfuming agent composed of the compound represented by Formula (I) according to the present invention can be used in combination with a fragrance(s) other than the compound represented by Formula (I), such as a commonly used other fragrance component or a fragrance blend having a desired composition. That is, a fragrance composition containing the perfuming agent composed of the compound represented by Formula (I) according to the present invention and a fragrance(s) other than the compound represented by Formula (I) is preferable, and also, a fragrance composition containing the perfuming agent composed of the compound represented by Formula (I) according to the present invention and a fragrance(s) other than the compounds represented by Formulae (I) and (II) is preferable. By using a fragrance(s) other than the compound represented by Formula (I) in combination, it is possible to impart, for example, a favorable fruity (apple-like), herbal, and minty odor to the fragrance composition of the present invention.

In the fragrance composition containing the compounds represented by Formulae (I) and (II) according to the present invention, nectar-like sweetness and voluminousness of the odor are imparted to the compound represented by Formula (II). The fragrance composition containing the compounds represented by Formulae (I) and (II) according to the present invention can be used in combination with a commonly used other fragrance component or a fragrance blend having a desired composition as the fragrance(s) other than the compounds represented by Formulae (I) and (II). By using a fragrance(s) other than the compounds represented by Formulae (I) and (II) in combination, it is possible to impart the odor of the compound represented by Formula (II) and nectar-like sweetness and voluminousness of the odor to the fragrance composition of the present invention. That is, the fragrance composition containing the compounds represented by Formulae (I) and (II) according to the present invention preferably further contains a fragrance(s) other than the compounds represented by Formulae (I) and (II).

Examples of the other fragrance(s) that can be used in combination with the perfuming agent composed of the compound represented by Formula (I) according to the present invention include fragrance components such as alcohols, hydrocarbons, phenols, esters, carbonates, aldehydes, ketones, acetals, ethers, carvones, lactones, nitriles, amines, Schiff bases, natural essential oils, and natural extracts (excluding the compound represented by Formula (I)). That is, the present invention relates to a fragrance composition containing: at least one substance that is other than compounds represented by Formulae (I) and (II) and is selected from alcohols, phenols, esters, carbonates, aldehydes, ketones, acetals, ethers, lactones, nitriles, amines, natural essential oils, and natural extracts: and the perfuming agent according to the present invention.

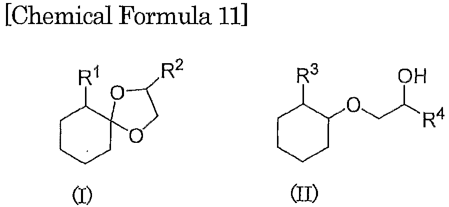

In Formulae (I) and (II),
R¹ and R³ are each independently an alkyl group having 1 or more and 5 or less of carbon atoms, and
R² and R⁴ are each independently a hydrogen atom or an alkyl group having 1 or more and 4 or less of carbon atoms.

The mass ratio between the perfuming agent composed of the compound represented by Formula (I) and a fragrance(s) other than the compounds represented by Formulae (I) and (II) (hereinafter simply referred to as the other fragrance(s)) (the compound represented by Formula (I)/the other fragrance(s)) is: preferably 0.01/99.99 or more, more preferably 0.1/99.9 or more, and still more preferably 1/99 or more from the viewpoint of allowing the perfuming agent to exhibit its function; preferably 1/2 or less, more preferably 1/3 or less, and still more preferably 1/4 or less from the same viewpoint; and preferably 0.01/99.99 or more and 1/2 or less, more preferably 0.1/99.9 or more and 1/3 or less, and still more preferably 1/99 or more and 1/4 or less.

The content of the perfuming agent composed of the compound represented by Formula (I) in the fragrance composition is as follows, from the viewpoint of allowing the perfuming agent to exhibit its function: preferably 0.01 mass% or more, more preferably 0.1 mass% or more, and still more preferably 0.2 mass% or more; preferably 20 mass% or less, more preferably 15 mass% or less, and still more preferably 10 mass% or less; and preferably 0.01 mass% or more and 20 mass% or less, more preferably 0.1 mass% or more and 15 mass% or less, and still more preferably 0.2 mass% or more and 10 mass% or less.

In the fragrance composition containing the compounds represented by Formulae (I) and (II) according to the present invention, examples of the other fragrance(s) that can be used in combination with the compounds represented by Formulae (I) and (II) include fragrance components such as alcohols, hydrocarbons, phenols, esters, carbonates, aldehydes, ketones, acetals, ethers, carvones, lactones, nitriles, amines, Schiff bases, natural essential oils, and natural extracts (excluding the compounds represented by Formulae (I) and (II)).

Of these, those preferably used for the above-described fragrance composition are alcohols, phenols, esters, carbonates, aldehydes, ketones, acetals, ethers, lactones, nitriles, amines, natural essential oils, and natural extracts.

Examples of the alcohols include aliphatic alcohols, terpene alcohols, aromatic alcohols, and other alcohols. Of these, aliphatic alcohols are preferable.

Examples of the terpene alcohols include linalool, ethyl linalool, citronellol, hydroxycitronellol, geraniol, tetrahydrogeraniol, nerol, terpineol, α-terpineol, dihydromyrcenol, farnesol, nerolidol, cedrol, menthol, and borneol.

Examples of the aromatic alcohols include phenylethyl alcohol, benzyl alcohol, dimethyl benzyl carbinol, phenylethyl dimethyl carbinol, and phenyl hexanol.

Examples of the aliphatic alcohols include cis-3-hexenol, 1-(2,2,6-trimethylcyclohexy)-3-hexanol, Sandahnysore Core (trade name, Kao Corporation, 2-methyl-4-(2,2,3-trimethyl-3-cyclopenten-1-yl)-2-buten-1-ol), Amber Core (trade name, Kao Corporation, 1-(2-t-butylcyclohexyloxy)-2-butanol), Magnol (trade name, Kao Corporation, a mixture containing ethyl norbornyl cyclohexanol as a main component), Undecavertol (trade name, Givaudan, 4-methyl-3-decen-5-ol), isobornyl cyclohexanol, and Terpirosa (trade name, Kao Corporation, 3,6-dimethyl-2-heptanol). It is to be noted here that, for "the fragrance composition containing the compounds represented by Formulae (I) and (II) according to the present invention", Amber Core is excluded from the above examples.

Examples of other alcohols include glycols such as dipropylene glycol, Florosa (trade name, Givaudan, chemical name:
4-methyl-2-(2-methylpropyl)tetrahydro-2H-4-pyranol), maltol, and ethyl maltol.

Examples of the hydrocarbons include limonene, α-pinene, β-pinene, terpinen, cedrene, longifolene, and valencene.

Examples of the phenols include guaiacol, eugenol, dihydroeugenol, isoeugenol, thymol, para-cresol, vanillin, and ethyl vanillin.

Examples of the esters include aliphatic carboxylic acid esters, aromatic carboxylic acid esters and other carboxylic acid esters.

Examples of aliphatic carboxylic acids that form the aliphatic carboxylic esters include straight-chain and branched carboxylic acids having 1 to 18 carbon atoms. Of these, carboxylic acids having 1 to 6 carbon atoms, such as formic acid, acetic acid, and propionic acid are important, and in particular, acetic acid is important. Examples of aromatic carboxylic acids that form the aromatic carboxylic esters include benzoic acid, anisic acid, phenylacetic acid, cinnamic acid, salicylic acid, and anthranilic acid. Examples of alcohols that form the aliphatic and aromatic esters include straight-chain and branched aliphatic alcohols having 1 to 5 carbon atoms and the alcohols described above as examples of the fragrance components.

Examples of formic acid esters include linalyl formate, citronellyl formate, and geranyl formate.

Examples of acetic acid esters include ethyl acetate, isoamyl acetate (isopentyl acetate), benzyl acetate, hexyl acetate, phenyl acetate, p-cresyl acetate, cis-3-hexenyl acetate, linalyl acetate, citronellyl acetate, geranyl acetate, neryl acetate, terpinyl acetate, nopyl acetate, bornyl acetate, isobornyl acetate, acetyl eugenol, acetyl isoeugenol, o-tert-butylcyclohexyl acetate, p-tert-butylcyclohexyl acetate, tricyclodecenyl acetate, phenylethyl acetate, styralyl acetate, cinnamyl acetate, dimethylbenzylcarbinyl acetate, 3-pentyltetrahydropyran-4-yl acetate, and prenyl acetate; and examples of phenylacetic acid esters include phenylethyl phenylacetate and p-cresyl phenylacetate.

Examples of propionic acid esters include ethyl propionate, citronellyl propionate, tricyclodecenyl propionate, benzyl propionate, styralyl propionate, and Helvetolide (trade name, Firmenich, 2-[1-(3,3-dimethylcyclohexyl)ethoxy]-2-methylpropyl)propionate).

Examples of butyric acid esters include citronellyl butyrate, dimethylbenzylcarbinyl n-butyrate, isoamyl n-butyrate, and n-amyl n-butyrate; and examples of isobutyric acid esters include tricyclodecenyl isobutyrate and phenoxyethyl isobutyrate.

Examples of valeric acid esters include methyl valerate, ethyl valerate, butyl valerate, amyl valerate, benzyl valerate, and phenylethyl valerate; and examples of hexanoic acid esters include methyl hexanoate, ethyl hexanoate, allyl hexanoate, linalyl hexanoate, and citronellyl hexanoate.

Examples of heptanoic acid esters include methyl heptanoate and allyl heptanoate.

Examples of nonenoic acid esters include methyl 2-nonenoate, ethyl 2-nonenoate, and ethyl 3-nonenoate.

Examples of benzoic acid esters include methyl benzoate, benzyl benzoate, and 3,6-dimethyl benzoate.

Examples of cinnamic acid esters include methyl cinnamate and benzyl cinnamate.

Examples of salicylic acid esters include methyl salicylate, n-hexyl salicylate, cis-3-hexenyl salicylate, cyclohexyl salicylate, and benzyl salicylate.

Examples of brassylic acid esters include ethylene brassylate.

Examples of tiglic acid esters include geranyl tiglate, 1-hexyl tiglate, and cis-3-hexenyl tiglate.

Examples of jasmonic acid esters include methyl jasmonate; and examples of dihydrojasmonic acid esters include MDJ (trade name, Kao Corporation, methyl dihydrojasmonate, methyl (2-pentyl-3-oxocyclopentyl)acetate).

Examples of glycidic acid esters include methyl 2,4-dihydroxy-ethyl methyl phenyl glycidate and 4-methyl phenyl ethyl glycidate.

Examples of anthranilic acid esters include methyl anthranilate, ethyl anthranilate, and dimethyl anthranilate.

Examples of glycolic acid esters include allylcyclohexyl glycolate.

Furthermore, examples of other esters include Ethyl Safranate (trade name, Givaudan, ethyl dihydrocyclogelanate), Poirenate (trade name, Kao Corporation, ethyl-2-cyclohexyl propionate), Fruitate (trade name, Kao Corporation, ethyl tricyclo[5.2.1.0^{2.6}]decane-2-carboxylate), ethyl acetoacetate, Fructone (trade name, IFF, ethyl 2-(2-methyl-1,3-dioxolan-2-yl)acetate), Manzanate (trade name, Givaudan, ethyl 2-methylpentanoate), ethyl 2-methylbutyrate, and allyl cyclohexylpropionate.

Examples of the carbonates include Liffarome (trade name, IFF, cis-3-hexenyl methyl carbonate), Jasmacyclat (trade name, Kao Corporation, methyl-cyclooctyl carbonate), and Floramat (trade name, Kao Corporation, ethyl-2-t-butylcyclohexyl carbonate).

Examples of the aldehydes include n-octanal, n-nonanal, n-decanal, n-dodecanal, 2-methylundecanal, 10-undecenal, citronellal, citral, hydroxycitronellal, Triplal (trade name, IFF, 2,4-dimethyl-3-cyclohexene-1-carboxyaldehyde), Cyclovertal (trade name, Kao Corporation, dimethyl-3-cyclohexenyl-1-carboxaldehyde), benzaldehyde, phenylacetaldehyde, phenylpropyl aldehyde, cinnamic aldehyde, dimethyltetrahydrobenzaldehyde, Bourgeonal (trade name, Givaudan, 3-(4-tert-butylphenyl)propanal), Lyral (trade name, IFF, hydroxy myrac aldehyde), Pollenal II (trade name, Kao Corporation, 2-cyclohexyl propanal), Lilial (trade name, Givaudan, p-tert-butyl-α-methylhydrocinnamic aldehyde), p-isopropyl-α-methylhydrocinnamic aldehyde, Floralozone (trade name, IFF, 3-(o-(and p-)ethylphenyl)-2,2-dimethylpropionaldehyde), α-amyl cinnamic aldehyde, α-hexyl cinnamic aldehyde, heliotropin, Helional (trade name, IFF, alpha-methyl-1,3-benzodioxole-5-propanal), Canthoxal (trade name, IFF, 2-methyl-3-(para-methoxyphenyl)propanal), and Aldehyde C-6 (trade name, Kao Corporation, n-hexanal).

Examples of the ketones include methylheptenone, dimethyloctenone, 3-octanone, hexylcyclopentanone, dihydrojasmone, Veloutone (trade name, Firmenich, 2,2,5-trimethyl-5-pentylcyclopentanone), Nectaryl (trade name, Givaudan, 2-(2-(4-methyl-3-cyclohexen-1-yl)propyl)cyclopentanone), ionone, β-ionone, methyl ionone, methyl ionone-G, γ-methyl ionone, damascene, α-damascone, β-damascone, δ-damascone, Isodamascone (trade name, Symrise, 1-(2,4,4-trimethyl-2-cyclohexyl)-trans-2-butanone), damascenone, Dynascone (trade name, Firmenich, 1-(5,5-dimethyl-1-cyclohexen-1-yl)-4-penten-1-one), irone, Cashmeran (trade name, IFF, 1,2,3,5,6,7-hexahydro-1,1,2,3,3-pentamethyl-4H-inden-4-one), Iso E Super (trade name, IFF, 1-(1,2,3,4,5,6,7,8-octahydro-2,3,8,8-tetramethyl-2-naphthalenyl)-ethan-1-one), Calone (trade name, Firmenich, 7-methyl-3,4-dihydro-2H-benzodioxepin-3-one), carvone, menthone, acetyl cedrene, isolongifolanone, nootkatone, benzylacetone, raspberry ketone, benzophenone, Tonalide (trade name, PFW, 6-acetyl-1,1,2,4,4,7-hexamethyltetrahydronaphthalene), 6-methylnaphthylketone, ethyl maltol, camphor, muscone, Muscenone (trade name, Firmenich, 3-methyl-5-cyclopentadecen-1-one), civetone, Glovanon (trade name, Symrise, 8-cyclohexadecenone), methylnonyl ketone, cis-jasmone, and para-amylcyclohexanone (4-pentyl cyclohexanone). Of these, ionone, damascenone, Iso E Super, or γ-methyl ionone is preferable from the viewpoint of enhancing floral sweetness when blended with other fragrances.

Examples of the acetals include acetaldehyde ethylphenylpropyl acetal, citral diethyl acetal, phenylacetaldehyde glyceryl acetal, ethyl acetoacetate ethylene glycol acetal, Boisambrene Forte (trade name, Kao Corporation, ethoxymethyl cyclododecyl ether), Troenan (trade name, Kao Corporation, 5-methyl-5-propyl-2-(1-methylbutyl)-1,3-dioxane), Floropal (trade name, Symrise, 2,4,6-trimethyl-4-phenyl-1,3-dioxane), and Magnolan (trade name, Symrise, 2,4-dimethyl-4,4a,5,9b-tetrahydroindeno[1,2-d][1,3]dioxane).

Examples of the ethers include cedryl methyl ether, estragole, anethole, β-naphthyl methyl ether, β-naphthyl ethyl ether, limonene oxide, rose oxide, nerol oxide, 1,8-cineole, rose furan, Ambroxan (trade name, Kao Corporation, [3aR-(3aα,5aβ,9aα,9bβ)]dodecahydro-3a,6,6,9a-tetramethylnaphtho[2,1-b]furan), Herbavert (trade name, Kao Corporation, 3,3,5-trimethylcyclohexyl ethyl ether), Galaxolide (trade name, IFF, hexamethylhexahydrocyclopentabenzopyran), and phenylacetaldehyde dimethylacetal.

Examples of the carboxylic acids include benzoic acid, phenylacetic acid, cinnamic acid, hydrocinnamic acid, butyric acid, and 2-hexenoic acid.

Examples of the lactones include ambrettolide, γ-decalactone, δ-decalactone, γ-valerolactone, γ-nonalactone, γ-undecalactone, 6-hexalactone, γ-jasmolactone, whisky lactone, coumarin, cyclopentadecanolide, cyclohexadecanolide, 11-oxahexadecanolide, and butylidene phthalide.

Examples of the nitriles include tridecene-2-nitrile, geranyl nitrile, citronellyl nitrile, and dodecanenitrile.

Examples of the amines include indole, skatole, 6-methylquinoline, 6-isopropylquinoline, and isobutylquinoline.

Examples of the Schiff bases include aurantiol and ligantraal.

Examples of the natural essential oils and the natural extracts include oils, concretes, absolutes, resinoids, oleoresins, tinctures, immersion liquids, and balsams of natural raw materials, such as orange, lemon, lime, bergamot, vanilla, mandarin, peppermint, spearmint, lavender, galbanum, chamomile, rosemary, eucalyptus, sage, basil, rose, rockrose, geranium, jasmine, ylang-ylang, anise, clove, ginger, nutmeg, cardamon, cedar, Japanese cypress, vetiver, patchouli, hay, lemongrass, labdanum, grapefruit, and elemi oils.

The contents of these other fragrances can be selected as appropriate according to, for example, the type of fragrance blend and the type and intensity of intended aromatic odor. The content of each of these other fragrances in a composition containing the fragrance composition of the present invention is preferably 0.0001 mass% or more and more preferably 0.001 mass% or more and is preferably 99.99 mass% or less and more preferably 80 mass% or less. The total content of the other fragrances in a composition containing the fragrance composition of the present invention is preferably 5 mass% or more and more preferably 50 mass% or more and is preferably 99.99 mass% or less and more preferably 99.95 mass% or less.

The mass ratio between the total amount of the compounds represented by Formulae (I) and (II) and the above-described other fragrance(s) (the total amount/the amount of the other fragrance(s)) is: preferably 0.01/99.99 or more, more preferably 0.1/99.9 or more, and still more preferably 1/99 or more from the viewpoint of allowing the perfuming agent to exhibit its function; preferably 1/2 or less, more preferably 1/3 or less, and still more preferably 1/4 or less from the same viewpoint; and preferably 0.01/99.99 or more and 1/2 or less, more preferably 0.1/99.9 or more and 1/3 or less, and still more preferably 1/99 or more and 1/4 or less.

The content of the compound represented by Formula (I) in the fragrance composition is as follows, from the viewpoint of allowing the perfuming agent to exhibit its function: preferably 0.01 mass% or more, more preferably 0.1 mass% or more, and still more preferably 0.2 mass% or more; preferably 20 mass% or less, more preferably 15 mass% or less, and still more preferably 10 mass% or less; and preferably 0.01 mass% or more and 20 mass% or less, more preferably 0.1 mass% or more and 15 mass% or less, and still more preferably 0.2 mass% or more and 10 mass% or less.

The fragrance composition of the present invention may be used in combination with an oil that serves as a base and itself is odorless. Such an oil allows fragrance components to be uniformly mixed together and to be easily added to a product, thereby allowing an odor with a moderate intensity to be more easily imparted to the product. Examples of the oil include: polyhydric alcohols such as ethylene glycol, propylene glycol, butylene glycol, and dipropylene glycol; esters such as isopropyl myristate, dibutyl adipate, and diethyl sebacate; hydrocarbons such as liquid paraffin and squalane; and surfactants such as polyoxyethylene alkyl ethers and sorbitan fatty acid esters.

Of these, polyhydric alcohols and esters are preferable as the oil from the viewpoint of solubility of all the fragrance components, and dipropylene glycol and isopropyl myristate are more preferable. The content of such an oil in the fragrance composition is preferably 0.01 mass% or more, more preferably 1 mass% or more, and still more preferably 5 mass% or more and is preferably 95 mass% or less, more preferably 90 mass% or less, and still more preferably 80 mass% or less.

The present invention further provides a cleaning composition containing the fragrance composition of the present invention, a cosmetic containing the fragrance composition of the present invention, and a fiber treating composition containing the fragrance composition of the present invention.

The cleaning composition of the present invention is preferably a body cleaning composition, a cleaning composition for clothing, or a cleaning composition for hard surfaces, more preferably a body cleaning composition or a cleaning composition for clothing, and still more preferably a cleaning composition for clothing.

The body cleaning composition is, for example, a skin cleaning composition, a hair cleaning composition, or a soap composition, and is preferably a skin cleaning composition.

The cleaning composition for hard surfaces is, for example, an all-purpose cleaner or a cleaning composition for tableware.

The fiber treating composition of the present invention is preferably a fabric softener composition.

The cosmetic of the present invention is preferably a perfume, a milky lotion, a skin lotion, or a sunscreen, and more preferably a perfume.

The cleaning composition of the present invention preferably contains an anionic surfactant in addition to the fragrance composition of the present invention, and may further contain a nonionic surfactant, a pH adjuster, a viscosity modifier, a solvent, an oil, an antiseptic agent, water, etc.

The fiber treating composition of the present invention preferably contains a cationic surfactant in addition to the fragrance composition of the present invention, and may further contain a pH adjuster, a solvent, an oil, an antiseptic agent, water, etc.

The perfume of the present invention may contain a solvent, water, or the like, in addition to the fragrance composition of the present invention.

The perfuming agent composed of the compound represented by Formula (I) and the fragrance composition containing the perfuming agent according to the present invention have a favorable fruity (apple-like), herbal, and minty odor. Further, the fragrance composition containing the compounds represented by Formulae (I) and (II) according to the present invention can create a new impression by imparting nectar-like sweetness and voluminousness of the odor to Amber Core.

Accordingly, the present invention relates to a method of using the perfuming agent of the present invention or the fragrance composition of the present invention ("the fragrance composition containing the perfuming agent composed of the compound represented by Formula (I) according to the present invention" and "the fragrance composition containing the compounds represented by Formulae (I) and (II) according to the present invention") as a fragrance-imparting component, and more specifically to a method of using the same as a fragrance-imparting component in a fragrance composition, a cleaning composition, a cosmetic, or a fiber treating composition. The cleaning composition is preferably a body cleaning composition, a cleaning composition for clothing, or a cleaning composition for hard surfaces, more preferably a body cleaning composition or a cleaning composition for clothing, and still more preferably a cleaning composition for clothing. The body cleaning composition is, for example, a skin cleaning composition, a hair cleaning composition, or a soap composition, and is preferably a skin cleaning composition. The cleaning composition for hard surfaces is, for example, an all-purpose cleaner or a cleaning composition for tableware. The cosmetic is preferably a perfume. The fiber treating composition is preferably a fabric softener composition.

In the above-described method of use, the fragrance composition of the present invention is used in an amount of preferably 0.0001 mass% or more and more preferably 0.001 mass% or more, and preferably 30 mass% or less, more preferably 20 mass% or less, still more preferably 10 mass% or less, 1 mass% or less, and even more preferably 0.1 mass% or less, with respect to the cleaning composition, the cosmetic, or the fabric softener composition. When the fragrance composition in an amount falling within such a range is formulated as a fragrance material to be blended with other components, it can create a new impression by causing a citrus-like top note having sparkling and lively impressions and also adding green and woody nuances to a floral odor, thus imparting naturalness as if in a flower shop to the floral odor.

In the above-described method of use, the fragrance composition of the present invention may be used in combination with an oil that itself is odorless. The oil is the same as that described above in connection with the fragrance composition. In the above-described method of use, the fragrance composition of the present invention may further contain, as another fragrance(s), a commonly used other fragrance component or a fragrance blend having a desired composition. Such other fragrances are the same as those described above in connection with the fragrance composition.

The present invention includes the following embodiments.
<1> A perfuming agent composed of a compound represented by Formula (I).

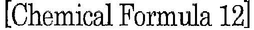

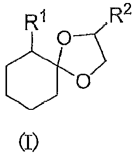

In Formula (I),
R¹ is an alkyl group having 1 or more and 5 or less of carbon atoms, and
R² is a hydrogen atom or an alkyl group having 1 or more and 4 or less of carbon atoms.

<2> The perfuming agent according to <1>,
   wherein R¹ is a tert-butyl group and R² is an ethyl group.
<3> A fragrance composition containing:
   at least one substance that is other than compounds represented by Formulae (I) and (II) and is selected from alcohols, phenols, esters, carbonates, aldehydes, ketones, acetals, ethers, lactones, nitriles, amines, natural essential oils, and natural extracts; and
   the perfuming agent according to <1> or <2>.

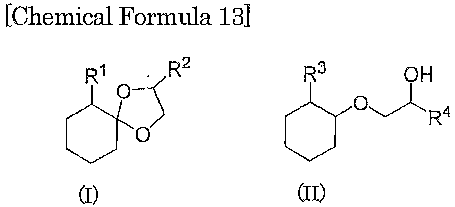

In Formulae (I) and (II),
R¹ and R³ are each independently an alkyl group having 1 or more and 5 or less of carbon atoms, and
R² and R⁴ are each independently a hydrogen atom or an alkyl group having 1 or more and 4 or less of carbon atoms.

<4> The fragrance composition according to <3>,
   wherein a mass ratio between the perfuming agent composed of the compound represented by Formula (I) and the at least one substance that is other than compounds represented by Formulae (I) and (II) and is selected from alcohols, phenols, esters, carbonates, aldehydes, ketones, acetals, ethers, lactones, nitriles, amines, natural essential oils, and natural extracts (hereinafter simply referred to as the other fragrance(s)) (the amount of the compound represented by Formula (I)/the total amount of the other fragrance(s)) is 0.01/99.99 or more and 1/2 or less.
<5> The fragrance composition according to <3> or <4>,
   wherein a mass ratio between the perfuming agent composed of the compound represented by Formula (I) and the at least one substance that is other than compounds represented by Formulae (I) and (II) and is selected from alcohols, phenols, esters, carbonates, aldehydes, ketones, acetals, ethers, lactones, nitriles, amines, natural essential oils, and natural extracts (hereinafter simply referred to as the other fragrance(s)) (the amount of the compound represented by Formula (I)/the total amount of the other fragrance(s)) is 0.1/99.9 or more and 1/3 or less.
<6> The fragrance composition according to any one of <3> to <5>,
   wherein a mass ratio between the perfuming agent composed of the compound represented by Formula (I) and the at least one substance that is other than compounds represented by Formulae (I) and (II) and is selected from alcohols, phenols, esters, carbonates, aldehydes, ketones, acetals, ethers, lactones, nitriles, amines, natural essential oils, and natural extracts (hereinafter simply referred to as the other fragrance(s)) (the amount of the compound represented by Formula (I)/the total amount of the other fragrance(s)) is 1/99 or more and 1/4 or less.
<7> The fragrance composition according to any one of <3> to <6>,
   wherein the content of the perfuming agent composed of the compound represented by Formula (I) in the fragrance composition is 0.01 mass% or more and 20 mass% or less.
<8> The fragrance composition according to any one of <3> to <7>,
   wherein the content of the perfuming agent composed of the compound represented by Formula (I) in the fragrance composition is 0.1 mass% or more and 15 mass% or less.
<9> The fragrance composition according to any one of <3> to <8>,
   wherein the content of the perfuming agent composed of the compound represented by Formula (I) in the fragrance composition is 0.2 mass% or more and 10 mass% or less.
<10> A cosmetic containing the fragrance composition according to any one of <3> to <9>.
<11> A cleaning composition containing the fragrance composition according to any one of <3> to <9>.
<12> A fiber treating agent composition containing the fragrance composition according to any one of <3> to <9>.
<13> A method of using the perfuming agent according to <1> or <2> as a fragrance-imparting component.
<14> A fragrance composition containing:
   a compound represented by Formula (II); and
   a compound represented by Formula (I),
   wherein a mass ratio between the compound represented by Formula (II) and the compound represented by Formula (I) (the compound represented by Formula (II) : the compound represented by Formula (I)) is 99.99:0.01 or more and 98:2 or less.

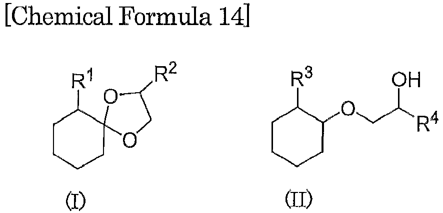

In Formulae (I) and (II),
R¹ and R³ are each independently an alkyl group having 1 or more and 5 or less of carbon atoms, and
R² and R⁴ are each independently a hydrogen atom or an alkyl group having 1 or more and 4 or less of carbon atoms.

<15> The fragrance composition according to <14>,
   wherein the mass ratio between the compound represented by Formula (II) and the compound represented by Formula (I) (the compound represented by Formula (II) : the compound represented by Formula (I)) is 99.99:0.01 or more and 99:1 or less.
<16> The fragrance composition according to <14> or <15>,
   wherein a mass ratio between the compound represented by Formula (II) and the compound represented by Formula (I) (the compound represented by Formula (II) : the compound represented by Formula (I)) is 99.97:0.03 or more and 99.20:0.80 or less.
<17> The fragrance composition according to any one of <14> to <16>,
   wherein a mass ratio between the compound represented by Formula (II) and the compound represented by Formula (I) (the compound represented by Formula (II) : the compound represented by Formula (I)) is 99.95:0.05 or more and 99.50:0.50 or less.
<18> The fragrance composition according to any one of <14> to <17>,
   wherein, in Formula (II), R³ is a tert-butyl group and R⁴ is an ethyl group.
<19> The fragrance composition according to any one of <14> to <18>,
   wherein, in Formulae (I) and (II), R¹ and R³ are tert-butyl groups and R² and R⁴ are ethyl groups.
<20> The fragrance composition according to any one of <14> to <19>, further containing at least one substance that is other than the compounds represented by Formulae (I) and (II) and is selected from alcohols, phenols, esters, carbonates, aldehydes, ketones, acetals, ethers, lactones, nitrile, amines, natural essential oils, and natural extracts.
<21> The fragrance composition according to any one of <14> to <20>,
   wherein a mass ratio between a total amount of the compounds represented by Formulae (I) and (II) and at least one substance that is other than the compounds represented by Formulae (I) and (II) and is selected from alcohols, phenols, esters, carbonates, aldehydes, ketones, acetals, ethers, lactones, nitriles, amines, natural essential oils, and natural extracts (hereinafter simply referred to as the other fragrance(s)) (the total amount of the compounds represented by Formulae (I) and (II)/the total amount of the other fragrance(s)) is 0.01/99.99 or more and 1/2 or less.
<22> The fragrance composition according to any one of <14> to <21>,
   wherein a mass ratio between a total amount of the compounds represented by Formulae (I) and (II) and at least one substance that is other than the compounds represented by Formulae (I) and (II) and is selected from alcohols, phenols, esters, carbonates, aldehydes, ketones, acetals, ethers, lactones, nitriles, amines, natural essential oils, and natural extracts (hereinafter simply referred to as the other fragrance(s)) (the total amount of the compounds represented by Formulae (I) and (II)/the total amount of the other fragrance(s)) is 0.1/99.9 or more and 1/3 or less.
<23> The fragrance composition according to any one of <14> to <22>,
   wherein a mass ratio between a total amount of the compounds represented by Formulae (I) and (II) and at least one substance that is other than the compounds represented by Formulae (I) and (II) and is selected from alcohols, phenols, esters, carbonates, aldehydes, ketones, acetals, ethers, lactones, nitriles, amines, natural essential oils, and natural extracts (hereinafter simply referred to as the other fragrance(s)) (the total amount of the compounds represented by Formulae (I) and (II)/the total amount of the other fragrance(s)) is 1/99 or more and 1/4 or less.
<24> The fragrance composition according to any one of <14> to <23>,
   wherein the content of the compound represented by Formula (I) in the fragrance composition is 0.01 mass% or more and 20 mass% or less.
<25> The fragrance composition according to any one of <14> to <24>,
   wherein the content of the compound represented by Formula (I) in the fragrance composition is 0.1 mass% or more and 15 mass% or less.
<26> The fragrance composition according to any one of <14> to <25>,
   wherein the content of the compound represented by Formula (I) in the fragrance composition is 0.2 mass% or more and 10 mass% or less.
<27> A cosmetic containing the fragrance composition according to any one of <14> to <26>.
<28> A cleaning composition containing the fragrance composition according to any one of <14> to <26>.
<29> A fiber treating agent composition containing the fragrance composition according to any one of <14> to <26>.
<30> A method of using the fragrance composition according to any one of <14> to <26> as a fragrance-imparting component.

In the following reference examples, examples, and comparative examples, "%" is "mass%", unless otherwise specified. The mass of a catalyst refers to the mass of the catalyst in a dry state.

### <Apparatus and Analytical Conditions for Gas Chromatography>

GC apparatus: 7890B, Agilent Technologies, Inc., hydrogen flame ionization detector
Column: For analysis of the yield, DB-1 (capillary column, 100% dimethylpolysiloxane, inner diameter: 0.25 mm, length: 30 m, film thickness: 0.25 µm, Agilent Technologies, Inc.) was used.

For analysis of a cis form and a trans form, DB-WAX (capillary column, polyethylene glycol, inner diameter: 0.25 mm, length: 30 m, film thickness: 0.25 µm, Agilent Technologies, Inc.) was used.
Carrier gas: He, 1.5 mL/min
Injection condition: 300°C, split ratio: 100/1
Injection Amount: 1 µL
Detection conditions: FID System, 300°C
Column temperature conditions: Starting at 80°C, the temperature was raised to 300°C at a rate of 10°C/min, and then maintained at 300°C for 10 minutes.

### Reference Example 1

### Production of 1-(2-t-butylphenyloxy)-2-butanol (3)

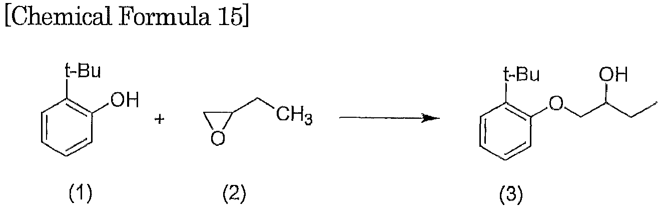

2-t-Butylphenol (1) (FUJIFILM Wako Pure Chemical Corporation, 350 g) and 35 g of 48% sodium hydroxide aqueous solution (Kanto Chemical Co., Inc.) were added to a 1-liter round-bottom flask provided with a Dimroth condenser and a dropping funnel in a nitrogen stream, and the resultant mixture was heated to 80°C. 1,2-Butylene oxide (2) (Tokyo Chemical Industry Co., Ltd., 176 g) was added dropwise thereto over about 2 hours, followed by stirring at 80°C for 5 hours. After cooling the reaction mixture, an organic layer was separated from the underlying sodium hydroxide aqueous solution and then distilled, whereby 1-(2-t-butylphenyloxy)-2 butanol (3) was obtained in a yield of 96%.

### Reference Example 2

### Production of 1-(2-t-butylcyclohexyloxy)-2-butanol (4)

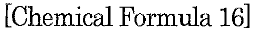

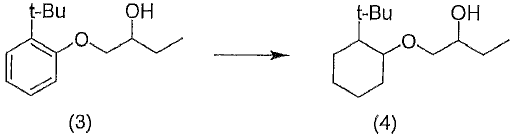

To a 500 ml autoclave, 1-(2-t-butylphenyloxy)-2-butanol (3) (250 g) obtained in Reference Example 1, an activated carbon-supported palladium catalyst (N.E. Chemcat Corporation, trade name: S-type, 50% water-containing product, palladium loading: 2%, 4.75 g), and an activated carbon-supported ruthenium catalyst (N.E. Chemcat Corporation, 50% water-containing product, ruthenium loading: 5%, 0.25 g) were added, and the resultant mixture was reacted for 6 hours at 190°C and at a hydrogen pressure of 2.0 MPa.

After completion of the reaction, the catalysts were filtered off and the filtrate was distilled, whereby 1-(2-t-butylcyclohexyloxy)-2-butanol (4) was obtained in a yield of 70%.

### Reference Example 3

### Production of 6-t-butyl-2-ethyl-1,4-dioxaspiro[4,5]decane (5)

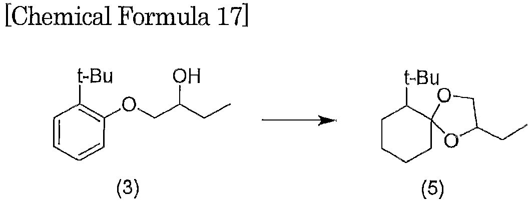

To a 500 ml autoclave, 1-(2-t-butylphenyloxy)-2-butanol (3) (250 g) obtained in Reference Example 1, an activated carbon-supported palladium catalyst (N.E. Chemcat Corporation, trade name: S'type, 50% water-containing product, palladium loading: 2%, 4.75 g), and an activated carbon-supported ruthenium catalyst (N.E. Chemcat Corporation, 50% water-containing product, ruthenium loading: 5%, 0.25 g) were added, and the resultant mixture was reacted for 2 hours at 190°C and at a hydrogen pressure of 2.0 MPa.

After completion of the reaction, the catalysts were filtered off and the filtrate was distilled, whereby 6-t-butyl-2-ethyl-1,4-dioxaspiro[4,5]decane (5) with a purity of 99.6% was obtained as a mixture of four diastereomers in a yield of 18%.

¹H-NMR (400 MHz, CDCl₃): 6(ppm) 0.8-1.1 (m, 12H), 1.1-2.0 (m, 11H), 3.3-3.7 (m, 1H), 3.9-4.2 (m, 2H)
IR (neat) : v (cm⁻¹) 511, 530, 592, 678, 750, 792, 808, 856, 866, 904, 930, 954, 975, 995, 1044, 1083, 1113, 1151, 1195, 1214, 1245, 1257, 1281, 1305, 1335, 1366, 1394, 1445, 1461, 1477, 2865, 2934.

### Example 1

6-t-Butyl-2-ethyl-1,4-dioxaspiro[4,5]decane (5) obtained in Reference Example 3 was subjected to sensory evaluation as a fragrance composition of Example 1 (the mass ratio of the compound (4) to the compound (5) = 0/100).

### Example 2

5 mg of 6-t-butyl-2-ethyl-1,4-dioxaspiro[4,5]decane (5) obtained in Reference Example 3 was added to and mixed with 50 g of 1-(2-t-butylcyclohexyloxy)-2-butanol (4) obtained in Reference Example 2 to obtain a fragrance composition (the mass ratio of the compound (4) to the compound (5) in the mixture = 99.99/0.01).

### Example 3

5 mg of 6-t-butyl-2-ethyl-1,4-dioxaspiro[4,5]decane (5) obtained in Reference Example 3 was added to and mixed with 10 g of 1-(2-t-butylcyclohexyloxy)-2-butanol (4) obtained in Reference Example 2 to obtain a fragrance composition (the mass ratio of the compound (4) to the compound (5) in the mixture = 99.95/0.05).

### Example 4

5 mg of 6-t-butyl-2-ethyl-1,4-dioxaspiro[4,5]decane (5) obtained in Reference Example 3 was added to and mixed with 5 g of 1-(2-t-butylcyclohexyloxy)-2-butanol (4) obtained in Reference Example 2 to obtain a fragrance composition (the mass ratio of the compound (4) to the compound (5) in the mixture = 99.90/0.10).

### Example 5

5 mg of 6-t-butyl-2-ethyl-1,4-dioxaspiro[4,5]decane (5) obtained in Reference Example 3 was added to and mixed with 1 g of 1-(2-t-butylcyclohexyloxy)-2-butanol (4) obtained in Reference Example 2 to obtain a fragrance composition (the mass ratio of the compound (4) to the compound (5) in the mixture = 99.50/0.50).

### Example 6

7.5 mg of 6-t-butyl-2-ethyl-1,4-dioxaspiro[4,5]decane (5) obtained in Reference Example 3 was added to and mixed with 1 g of 1-(2-t-butylcyclohexyloxy)-2-butanol (4) obtained in Reference Example 2 to obtain a fragrance composition (the mass ratio of the compound (4) to the compound (5) in the mixture = 99.26/0.74).

### Example 7

10 mg of 6-t-butyl-2-ethyl-1,4-dioxaspiro[4,5]decane (5) obtained in Reference Example 3 was added to and mixed with 1 g of 1-(2-t-butylcyclohexyloxy)-2-butanol (4) obtained in Reference Example 2 to obtain a fragrance composition (the mass ratio of the compound (4) to the compound (5) in the mixture = 99.01/0.99).

### Comparative Example 1

1-(2-t-Butylcyclohexyloxy)-2-butanol (4) obtained in Reference Example 2 was subjected to sensory evaluation as a fragrance composition of Comparative Example 1 (the mass ratio of the compound (4) to the compound (5) = 100/0).

### Example 8

30 mg of 6-t-butyl-2-ethyl-1,4-dioxaspiro[4,5]decane (5) obtained in Reference Example 3 was added to and mixed with 1 g of 1-(2-t-butylcyclohexyloxy)-2-butanol (4) obtained in Reference Example 2 to obtain a fragrance composition (the mass ratio of the compound (4) to the compound (5) in the mixture = 97.09/2.91).

### Example 9

50 mg of 6-t-butyl-2-ethyl-1,4-dioxaspiro[4,5]decane (5) obtained in Reference Example 3 was added to and mixed with 1 g of 1-(2-t-butylcyclohexyloxy)-2-butanol (4) obtained in Reference Example 2 to obtain a fragrance composition (the mass ratio of the compound (4) to the compound (5) = 95.2414.76).

### Sensory Evaluation

The odor of each of the fragrance compositions obtained in Examples 2 to 9 and Comparative Example 1 were evaluated by three expert panelists. The evaluation was made using a fragrance test paper (Daimonji Paper Co., Ltd., 150 mm × 7 mm). The fragrance compositions of Examples 1 to 9 and Comparative Example 1, which differ from each other in the ratio of the isomers, were each applied to the tip of the fragrance test paper, and the evaluation was performed indoor after one hour. Sensory evaluation was made in terms of odor characteristics and odor intensity. The odor characteristics and odor intensity were relatively evaluated on a scale of 1 to 5, with higher numbers representing better results. For odor persistence, how many days the odor would last was examined, and the number of days the odor had lasted was recorded. In the sensory evaluation, the three expert panelists also described how they had felt about the quality and characteristics of the overall odor. The results obtained are shown in Table 1.

### Evaluation of Example 1

The evaluation of the odor of the fragrance composition obtained in Example 1 (6-t-butyl-2-ethyl-1,4-dioxaspiro[4,5]decane alone) revealed that this fragrance composition had a favorable aromatic odor as a fragrance with fruity, apple-like, and nectar-like sweetness together with slightly herbal, minty, and woody aspects. None of the panelists perceived a pungent chemical odor or a tobacco- or leather-like aromatic odor, and this confirmed that this fragrance composition is widely usable in preparation of fragrance blends.

**[Table 1]**

| | | | | | |
|---|---|---|---|---|---|
| 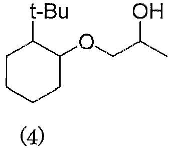 | | | 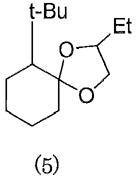 | | |

| | Compound (4) : compound (5) mass ratio | Amber, woody characteristics | Sweetness | Voluminousness | Odor evaluation (by three expert panelists) |
|---|---|---|---|---|---|
| Comp. Ex. 1 | 100:0 | 5 | 1 | 2 | Strong amber like, woody, and slight camphor-like odor |
| | | | | | The odor lacks in nectar-like sweetness and voluminousness. |
| Ex. 2 | 99.99:0.01 | 5 | 3 | 4 | Strong amber like, woody, and slight camphor-like odor |
| | | | | | The odor has nectar like sweetness imparted thereto as well as voluminousness. |
| Ex. 3 | 99.95:0.05 | 5 | 5 | 5 | Strong amber like, woody, and slight camphorlike odor |
| | | | | | The odor has nectar like sweetness imparted thereto as well as voluminousness, and is particularly well-balanced. |
| Ex. 4 | 99.90:0.10 | 5 | 5 | 5 | Strong amber like, woody, and slight camphor-like odor |
| | | | | | The odor has nectar like sweetness imparted thereto as well as voluminousness, and is particularly well-balanced. |
| Ex. 5 | 99.50:0.50 | 5 | 5 | 5 | Strong amber like, woody, and slight camphorlike odor |
| | | | | | The odor has nectar-like sweetness imparted thereto as well as voluminousness, and is particularly well-balanced. |
| Ex. 6 | 99.26:0.74 | 4 | 5 | 4 | Strong amber like, woody, and slight camphor-like odor. |
| | | | | | The odor has nectar-like sweetness imparted thereto as well as voluminousness, and is well-balanced. |
| Ex. 7 | 99.01:0.99 | 3 | 5 | 4 | Strong amber like, woody, and slight camphor-like odor |
| | | | | | The odor has nectarlike sweetness imparted thereto as well as voluminousness. |
| Ex. 8 | 97.09:2.91 | 1 | 5 | 2 | Amber-like, woody, and slight camphor-like odor |
| | | | | | Honey-like fruity sweetness seems a little too strong, and the odor lacks in balance. |
| Ex. 9 | 95.24:4.76 | 1 | 5 | 1 | The original amber-like and woody odor characteristics seem to be less perceivable. Honey-like fruity sweetness seems too strong, which disturbs the balance of the odor. |

In Table 1, the term "voluminousness" means the intensity, profoundness, and richness of odors, and it is thus preferable for a fragrance to have voluminousness. As can be seen from Table 1, the fragrance compositions of the present invention each exhibited an effect of imparting nectar-like sweetness and voluminousness of the odor while maintaining a strong amber-like, woody, and slight camphor-like aromatic odor.

### Example 10: Blend Example 1

6-t-Butyl-2-ethyl-1,4-dioxaspiro[4,5]decane (5) obtained in Reference Example 3 was added to 900 parts by mass of a jasmine-note fragrance blend with the following composition, and the resulting composition was subjected to sensory evaluation. The sensory evaluation was performed in the same manner as in Examples 1 to 9 and Comparative Example 1. The results obtained are shown in Table 4. Regarding the results of the evaluation, the degree of effectiveness was relatively evaluated on a scale of 1 to 5, with higher numbers representing better results.

**[Table 2]**

| <Composition of Jasmine Fragrance Blend> | |
|---|---|
| γ-Undecalactone | 10 parts by mass |
| Allyl heptanoate | 20 parts by mass |
| Benzyl acetate | 100 parts by mass |
| Benzyl salicylate | 20 parts by mass |
| β-Pinene | 40 parts by mass |
| cis-3-Hexenol | 0.2 parts by mass |
| Dihydrojasmone | 3 parts by mass |
| Ethyl linalool | 50 parts by mass |
| Fructone¹⁾ | 50 parts by mass |
| Indole | 0.01 parts by mass |
| γ-Decalactone | 3 parts by mass |
| Linalool | 150 parts by mass |
| Manzanate²⁾ | 4 parts by mass |
| MDJ³⁾ | 250 parts by mass |
| p-Cresyl acetate | 0.01 parts by mass |
| Styralyl acetate | 15 parts by mass |
| Triplal⁴⁾ | 3 parts by mass |
| Dipropylene glycol | 181.78 parts by mass |
| Total | 900 parts by mass |

| | |
|---|---|
| 1) Fructone: trade name, IFF, ethyl 2-(2-methyl-1,3-dioxolan-2-yl)acetate 2) Manzanate: trade name, Givaudan, ethyl 2-methylpentanoate 3) MDJ: trade name, Kao Corporation, methyl dihydrojasmonate, methyl (2-pentyl-3-oxocyclopentyl)acetate 4) Triplal: trade name, IFF, 2,4-dimethyl-3-cyclohexene-1-carboxyaldehyde | |

**[Table 3]**

| <Composition of Blend Example> | | | | |
|---|---|---|---|---|
| | Jasmine fragrance blend in Table 2 | Dipropylene glycol | Compound (5) | Total |
| Comp. Ex. 2 | 900 parts by mass | 100 parts by mass | - | 1000 parts by mass |
| Ex. 10 | 900 parts by mass | - | 100 parts by mass | 1000 parts by mass |

**[Table 4]**

| <Results of Sensory Evaluation> | | |
|---|---|---|
| | Floral odor | Odor evaluation (by three expert panelists) |
| Comp. Ex. 2 | 3 | The composition has a jasmine-like floral odor, but lacks in naturalness and voluminousness of the overall odor. |
| Ex. 10 | 5 | The composition has a jasmine-like floral odor with nectar-like sweetness added, and the overall odor is in harmony. White floral top and middle notes are emphasized, and the naturalness and the voluminousness of the overall odor are enhanced. |

As can be seen from Table 4, the results of the sensory evaluation by the three expert panelists revealed that the fragrance composition of the present invention imparted nectar-like sweetness to the jasmine-like fragrance blend and enhanced the naturalness and the voluminousness of the overall odor.

### Example 11: Blend Example 2

6-t-Butyl-2-ethyl-1,4-dioxaspiro[4,5]decane (5) obtained in Reference Example 3 was added to 925 parts by mass of a pear-note fragrance blend with the following composition, and the resulting composition was subjected to sensory evaluation. The sensory evaluation was performed in the same manner as in Examples 1 to 10 and Comparative Examples 1 and 2. The results obtained are shown in Table 7. Regarding the results of the evaluation, the degree of effectiveness was relatively evaluated on a scale of 1 to 5, with higher numbers representing better results.

**[Table 5]**

| <Composition of Pear Fragrance Blend> | |
|---|---|
| α-Terpineol | 10 parts by mass |
| Cashmeran¹⁾ | 5 parts by mass |
| cis-3-Hexenyl acetate | 5 parts by mass |
| cis-3-Hexenyl salicylate | 10 parts by mass |
| Geraniol | 100 parts by mass |
| Hexyl acetate | 100 parts by mass |
| Maltol | 0.1 parts by mass |
| Poirenate²⁾ | 200 parts by mass |
| Dipropylene glycol | 494.9 parts by mass |
| Total | 925 parts by mass |

| | |
|---|---|
| 1) Cashmeran: trade name, IFF, 1,2,3,5,6,7-hexahydro-1,1,2,3, 3-pentamethyl-4H-inden-4-one 2) Poirenate: trade name, Kao Corporation, ethyl 2-cyclohexyl propionate | |

**[Table 6]**

| <Composition of Blend Example> | | | | |
|---|---|---|---|---|
| | Pear fragrance blend in Table 5 | Dipropylene glycol | Compound (5) | Total |
| Comp. Ex. 3 | 925 parts by mass | 75 parts by mass | - | 1000 parts by mass |
| Ex. 11 | 925 parts by mass | - | 75 parts by mass | 1000 parts by mass |

**[Table 7]**

| <Results of Sensory Evaluation> | | |
|---|---|---|
| | Fruity odor | Odor evaluation (by three expert panelists) |
| Comp. Ex. 3 | 3 | The composition has a pear-like fruity odor, but lacks in naturalness and voluminousness of the overall odor. |
| Ex. 11 | 5 | The composition has a pear-like fruity odor with emphasis on a pear-like green odor and a fruity odor, and the naturalness and the voluminousness of the overall odor are enhanced. |

As can be seen from Table 7, the results of the sensory evaluation by the three expert panelists revealed that the fragrance composition of the present invention enhanced the naturalness and the voluminousness of the overall odor of the pear-like fragrance blend.

### Example 12: Blend Example 3

6-t-Butyl-2-ethyl-1,4-dioxaspiro[4,5]decane (5) obtained in Reference Example 3 was added to 970 parts by mass of a lime-note fragrance blend with the following composition, and the resulting composition was subjected to sensory evaluation. The sensory evaluation was performed in the same manner as in Examples 1 to 11 and Comparative Examples 1 to 3. The results obtained are shown in Table 10. Regarding the results of the evaluation, the degree of effectiveness was relatively evaluated on a scale of 1 to 5, with higher numbers representing better results.

**[Table 8]**

| <Composition of Lime Fragrance Blend> | |
|---|---|
| cis-3-Hexenyl salicylate | 30 parts by mass |
| Coumarin | 0.5 parts by mass |
| Ethylene brassylate | 100 parts by mass |
| Galbanum oil | 0.1 parts by mass |
| Geraniol | 50 parts by mass |
| Geranium resinoid 10% IPM | 1 part by mass |
| Geranyl acetate | 20 parts by mass |
| Hay absolute | 0.2 parts by mass |
| Lime terpene | 50 parts by mass |
| Limonene | 150 parts by mass |
| MDJ¹⁾ | 200 parts by mass |
| Terpineol | 20 parts by mass |
| Dipropylene glycol | 348.2 parts by mass |
| Total | 970 parts by mass |

| | |
|---|---|
| 1) MDJ: trade name, Kao Corporation, methyl dihydrojasmonate, methyl (2-pentyl-3-oxocyclopentyl)acetate | |

**[Table 9]**

| <Composition of Blend Example> | | | | |
|---|---|---|---|---|
| | Lime fragrance blend in Table 8 | Dipropylene glycol | Compound (5) | Total |
| Comp. Ex. 4 | 970 parts by mass | 30 parts by mass | - | 1000 parts by mass |
| Ex. 12 | 970 parts by mass | - | 30 parts by mass | 1000 parts by mass |

**[Table 10]**

| <Results of Sensory Evaluation> | | |
|---|---|---|
| | Citrus odor | Odor evaluation (by three expert panelists) |
| Comp. Ex. 4 | 3 | The composition has a lime-like citrus odor, but its green odor is weak and it lacks in balance and naturalness. |
| Ex. 12 | 5 | The composition has a lime-like citrus odor with emphasis on a lime-like green odor. The overall odor is in harmony, and has naturalness imparted thereto. |

As can be seen from Table 10, the results of the sensory evaluation by the three expert panelists revealed that the fragrance composition of the present invention emphasized the lime-like green odor, brought the overall odor into harmony, and imparted naturalness to the lime-like fragrance blend.

### Example 13: Blend Example 4

6-t-Butyl-2-ethyl-1,4-dioxaspiro[4,5]decane (5) obtained in Reference Example 3 was added to 950 parts by mass of a rose-note fragrance blend with the following composition, and the resulting composition was subjected to sensory evaluation. The sensory evaluation was performed in the same manner as in Examples 1 to 12 and Comparative Examples 1 to 4. The results obtained are shown in Table 13. Regarding the results of the evaluation, the degree of effectiveness was relatively evaluated on a scale of 1 to 5, with higher numbers representing better results.

**[Table 11]**

| <Composition of Rose Fragrance Blend> | |
|---|---|
| Allyl heptanoate | 10 parts by mass |
| Ambroxan¹⁾ | 1 part by mass |
| Citral | 15 parts by mass |
| Damascenone | 1 part by mass |
| Eugenol | 5 parts by mass |
| Geraniol | 250 parts by mass |
| Hydroxycitronellol | 50 parts by mass |
| Hydroxycitronellal | 25 parts by mass |
| γ-Decalactone | 10 parts by mass |
| Lilial²⁾ | 120 parts by mass |
| Linalool | 30 parts by mass |
| Nerol | 30 parts by mass |
| Phenylethyl alcohol | 400 parts by mass |
| Tetrahydrogeraniol | 3 parts by mass |
| Total | 950 parts by mass |

| | |
|---|---|
| 1) Ambroxan: trade name, Kao Corporation, [3aR-(3aα,5aβ,9aα,9bβ)]dodecahydro-3a,6,6,9a-tetramethylnaphtho[2,1-b]furan 2) Lilial: trade name, Givaudan, p-tert-butyl-α-methylhydrocinnamic aldehyde | |

**[Table 12]**

| <Composition of Blend Example> | | | | |
|---|---|---|---|---|
| | Rose fragrance blend in Table 11 | Dipropylene glycol | Compound (5) | Total |
| Comp. Ex. 5 | 950 parts by mass | 50 parts by mass | - | 1000 parts by mass |
| Ex. 13 | 950 parts by mass | - | 50 parts by mass | 1000 parts by mass |

**[Table 13]**

| <Results of Sensory Evaluation> | | |
|---|---|---|
| | Floral odor | Odor evaluation (by three expert panelists) |
| Comp. Ex. 5 | 3 | The composition has a rose-like floral odor, but lacks in naturalness and voluminousness of the overall odor. |
| Ex. 13 | 5 | The composition has a rose-like floral odor with sweetness imparted thereto, and rosy-tone naturalness and voluminousness of the overall odor are enhanced. |

As can be seen from Table 13, the results of the sensory evaluation by the three expert panelists revealed that the fragrance composition of the present invention imparted sweetness and enhanced the naturalness and the voluminousness of the overall odor of the rose-like fragrance blend.

### Example 14: Blend Example 5

6-t-Butyl-2-ethyl-1,4-dioxaspiro[4,5]decane (5) obtained in Reference Example 3 was added to 900 parts by mass of a lychee-note fragrance blend with the following composition, and the resulting composition was subjected to sensory evaluation. The sensory evaluation was performed in the same manner as in Examples 1 to 13 and Comparative Examples 1 to 5. The results obtained are shown in Table 16. Regarding the results of the evaluation, the degree of effectiveness was relatively evaluated on a scale of 1 to 5, with higher numbers representing better results.

**[Table 14]**

| <Composition of Lychee Fragrance Blend> | |
|---|---|
| Citronellol | 150 parts by mass |
| Dimethylbenzylcarbinyl n-butyrate | 50 parts by mass |
| Damascenone | 2.4 parts by mass |
| Ethyl propionate | 30 parts by mass |
| Ethylene brassylate | 50 parts by mass |
| Floropal¹⁾ | 10 parts by mass |
| Galaxolide²⁾ 70BB | 80 parts by mass |
| Geraniol | 100 parts by mass |
| Heliotropin | 30 parts by mass |
| α-Hexyl cinnamic aldehyde | 70 parts by mass |
| β-Ionone | 30 parts by mass |
| Isoamyl n-butyrate | 20 parts by mass |
| γ-Decalactone | 2.4 parts by mass |
| Lilial³⁾ | 30 parts by mass |
| Linalool | 30 parts by mass |
| Magnolan⁴⁾ | 30 parts by mass |
| Maltol | 0.2 parts by mass |
| n-Amyl n-butyrate | 10 parts by mass |
| Phenoxyethyl isobutyrate | 30 parts by mass |
| Poirenate⁵⁾ | 70 parts by mass |
| Prenyl acetate | 10 parts by mass |
| Rose oxide | 5 parts by mass |
| Troenan⁶⁾ | 20 parts by mass |
| Dipropylene glycol | 40 parts by mass |
| Total | 900 parts by mass |

| | |
|---|---|
| 1) Floropal: trade name, Symrise, 2,4,6-trimethyl-4-phenyl-1,3-dioxane 2) Galaxolide: trade name, IFF, hexamethylhexahydrocyclopentabenzopyran 3) Lilial: trade name, Givaudan, p-tert-butyl-α-methylhydrocinnamic aldehyde 3) Magnolan: trade name, Symrise, 2,4-dimethyl-4,4a,5,9b-tetrahydroindeno[1,2-d][1,3]dioxane 4) Poirenate: trade name, Kao Corporation, ethyl 2-cyclohexyl propionate 5) Troenan: trade name, Kao Corporation, 5-methyl-5-propyl-2-(1-methylbutyl)-1,3-dioxane | |

**[Table 15]**

| <Composition of Blend Example> | | | | |
|---|---|---|---|---|
| | Lychee fragrance blend in Table 14 | Dipropylene glycol | Compound (5) | Total |
| Comp. Ex. 6 | 900 parts by mass | 100 parts by mass | - | 1000 parts by mass |
| Ex. 14 | 900 parts by mass | - | 100 parts by mass | 1000 parts by mass |

**[Table 16]**

| <Results of Sensory Evaluation> | | |
|---|---|---|
| | Fruity odor | Odor evaluation (by three expert panelists) |
| Comp. Ex. 6 | 3 | The composition has a lychee-like fruity odor, but has poor emanation of the odor and lacks sweetness and voluminousness of the overall odor. |
| Ex. 14 | 5 | The composition has a lychee-like fruit odor with emphasis on emanation of a lychee-like odor and on a green odor. Fruit flesh-like sweetness is enhanced, and voluminousness is imparted to the odor. |

As can be seen from Table 16, the results of the sensory evaluation by the three expert panelists revealed that the fragrance composition of the present invention emphasized emanation of the odor and imparted sweetness and voluminousness of the odor to the lychee-like fragrance blend.

### Example 15: Blend Example 6

6-t-Butyl-2-ethyl-1,4-dioxaspiro[4,5]decane (5) obtained in Reference Example 3 was added to 900 parts by mass of a fougere-note fragrance blend with the following composition, and the resulting composition was subjected to sensory evaluation. The sensory evaluation was performed in the same manner as in Examples 1 to 14 and Comparative Examples 1 to 6. The results obtained are shown in Table 19. Regarding the results of the evaluation, the degree of effectiveness was relatively evaluated on a scale of 1 to 5, with higher numbers representing better results.

**[Table 17]**

| <Composition of Fougere Fragrance Blend> | |
|---|---|
| Ambroxan¹⁾ | 10 parts by mass |
| Bergamot oil BGF | 15 parts by mass |
| cis-3-Hexenyl salicylate | 50 parts by mass |
| Coumarin | 1 part by mass |
| Ethylene brassylate | 60 parts by mass |
| Geranium oil | 5 parts by mass |
| Heliotropin | 2.5 parts by mass |
| Helvetolide²⁾ | 30 parts by mass |
| Lavender MB40/42 | 5 parts by mass |
| Lemon Italy BGF | 20 parts by mass |
| Linalyl acetate | 30 parts by mass |
| MDJ³⁾ | 200 parts by mass |
| γ-Methyl ionone | 4 parts by mass |
| Rosemary absolute | 0.2 parts by mass |
| Sage clary oil | 20 parts by mass |
| Dipropylene glycol | 447.3 parts by mass |
| Total | 900 parts by mass |

| | |
|---|---|
| 1) Ambroxan: trade name, Kao Corporation, [3aR-(3aα,5aβ,9aα,9bβ)]dodecahydro-3a,6,6,9a-tetramethylnaphtho[2,1-b]furan 2) Helvetolide: trade name, Firmenich, 2-[1-(3,3-dimethylcyclohexyl)ethoxy]-2-methylpropyl)propionate 3) MDJ: trade name, Kao Corporation, methyl dihydrojasmonate, methyl (2-pentyl-3-oxocyclopentyl)acetate | |

**[Table 18]**

| <Composition of Blend Example> | | | | |
|---|---|---|---|---|
| | Fougere fragrance blend in Table 17 | Dipropylene glycol | Compound (5) | Total |
| Comp. Ex. 7 | 900 parts by mass | 100 parts by mass | - | 1000 parts by mass |
| Ex. 15 | 900 parts by mass | - | 100 parts by mass | 1000 parts by mass |

**[Table 19]**

| <Results of Sensory Evaluation> | | |
|---|---|---|
| | Fougere odor | Odor evaluation (by three expert panelists) |
| Comp. Ex. 7 | 3 | The composition has an odor of a fougere-tone perfume, but lacks in glamorous and powdery impressions and also lacks in the voluminousness of the overall odor. |
| Ex. 15 | 5 | The composition has an odor of a fougere-tone perfume with emphasis on glamorous and powdery impressions of a fougere-tone odor and also has voluminousness imparted thereto. |

As can be seen from Table 19, the results of the sensory evaluation by the three expert panelists revealed that the fragrance composition of the present invention emphasized the glamorous and powdery impressions and imparted sweetness and voluminousness of the odor to the fougere-tone fragrance blend.

### Example 16: Blend Example 7

1-(2-t-butylcyclohexyloxy)-2-butanol (4) obtained in Reference Example 2 and 6-t-butyl-2-ethyl-1,4-dioxaspiro[4,5]decane (5) obtained in Reference Example 3 were added to 730 parts by mass of a green apple-note fragrance blend with the following composition, and the resulting composition was subjected to sensory evaluation. The sensory evaluation was performed in the same manner as in Examples 1 to 15 and Comparative Examples 1 to 7. The results obtained are shown in Table 22. Regarding the results of the evaluation, the degree of effectiveness was relatively evaluated on a scale of 1 to 5, with higher numbers representing better results.

**[Table 20]**

| <Composition of Green Apple Fragrance Blend> | |
|---|---|
| Aldehyde C-6¹⁾ | 30 parts by mass |
| Hexyl acetate | 200 parts by mass |
| Ethyl 2-methylbutyrate | 100 parts by mass |
| γ-Decalactone | 50 parts by mass |
| Ethyl acetoacetate | 100 parts by mass |
| Phenyl acetate | 100 parts by mass |
| Terpirosa²⁾ | 150 parts by mass |
| Total | 730 parts by mass |

| | |
|---|---|
| 1) Aldehyde C-6: trade name, Kao Corporation, n-hexanal 2) Terpirosa: trade name, Kao Corporation, 3,6-dimethyl-2-heptanol | |

**[Table 21]**

| <Composition of Blend Example> | | | | | |
|---|---|---|---|---|---|
| | Green apple fragrance blend in Table 20 | Dipropylene glycol | Compound (4) | Compound (5) | Total |
| Comp. Ex. 8 | 730 parts by mass | 270 parts by mass | - | - | 1000 parts by mass |
| Comp. Ex. 9 | 730 parts by mass | - | 270 parts by mass | - | 1000 parts by mass |
| Ex. 16 | 730 parts by mass | - | 268 parts by mass | 2 parts by mass | 1000 parts by mass |

**[Table 22]**

| <Results of Sensory Evaluation> | | |
|---|---|---|
| | Fruity odor | Odor evaluation (by three expert panelists) |
| Comp. Ex. 8 | 3 | The composition has a green apple-like fruit odor, but lacks in freshness, fruity sweetness, and voluminousness. |
| Comp. Ex. 9 | 4 | Although green apple-like, fresh, and fruity sweetness is perceived, the odor lacks in voluminousness. |
| Ex. 16 | 5 | Green apple-like, fresh, and fruity sweetness and also voluminousness are strongly perceived. |

As can be seen from Table 22, the results of the sensory evaluation by the three expert panelists revealed that the fragrance composition of the present invention newly imparted freshness and fruity sweetness and also imparted voluminousness to the fruity fragrance blend.

The perfuming agent containing the compound represented by Formula (I) and the fragrance composition containing the perfuming agent according to the present invention can provide fragrance compositions with a favorable fruity (apple-like), herbal, and minty odor. Further, the fragrance composition containing the compounds of Formulae (I) and (II) imparts nectar-like sweetness and voluminousness of the odor to Amber Core.

## Claims

1. A perfuming agent composed of a compound represented by Formula (I): 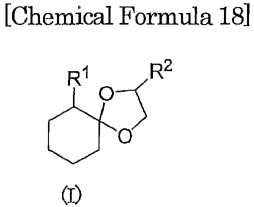 where
R¹ is an alkyl group having 1 or more and 5 or less of carbon atoms, and
R² is a hydrogen atom or an alkyl group having 1 or more and 4 or less of carbon atoms.

2. The perfuming agent according to claim 1,
wherein R¹ is a tert-butyl group, and
R² is an ethyl group.

3. A fragrance composition comprising:
at least one substance that is other than compounds represented by Formulae (I) and (II) and is selected from alcohols, phenols, esters, carbonates, aldehydes, ketones, acetals, ethers, lactones, nitriles, amines, natural essential oils, and natural extracts; and
the perfuming agent according to claim 1 or 2: 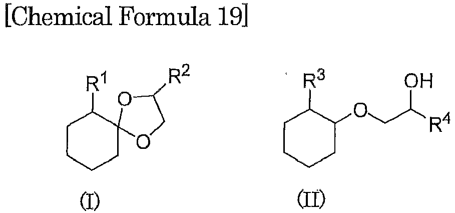
where
R¹ and R³ are each independently an alkyl group having 1 or more and 5 or less of carbon atoms, and
R² and R⁴ are each independently a hydrogen atom or an alkyl group having 1 or more and 4 or less of carbon atoms.

4. A cosmetic comprising the fragrance composition according to claim 3.

5. A cleaning composition comprising the fragrance composition according to claim 3.

6. A fiber treating agent composition comprising the fragrance composition according to claim 3.

7. A method of using the perfuming agent according to claim 1 or 2 as a fragrance-imparting component.

8. A fragrance composition comprising:
a compound represented by Formula (II); and
a compound represented by Formula (I),
wherein a mass ratio between the compound represented by Formula (II) and the compound represented by Formula (I) (the compound represented by Formula (II) : the compound represented by Formula (I)) is 99.99:0.01 or more and 98:2 or less: 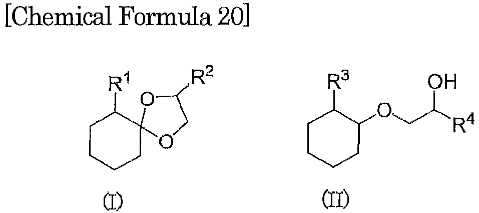
where
R¹ and R³ are each independently an alkyl group having 1 or more and 5 or less of carbon atoms, and
R² and R⁴ are each independently a hydrogen atom or an alkyl group having 1 or more and 4 or less of carbon atoms.

9. The fragrance composition according to claim 8,
wherein the mass ratio between the compound represented by Formula (II) and the compound represented by Formula (I) (the compound represented by Formula (II) : the compound represented by Formula (I)) is 99.97:0.03 or more and 99.20:0.80 or less.

10. The fragrance composition according to claim 8 or 9,
wherein, in Formula (II), R³ is a tert-butyl group and R⁴ is an ethyl group.

11. The fragrance composition according to any one of claims 8 to 10,
wherein, in Formulae (I) and (II), R¹ and R³ are tert-butyl groups and R² and R⁴ are ethyl groups.

12. The fragrance composition according to any one of claims 8 to 11, further comprising at least one substance that is other than the compounds represented by Formulae (I) and (II) and is selected from alcohols, phenols, esters, carbonates, aldehydes, ketones, acetals, ethers, lactones, nitriles, amines, natural essential oils, and natural extracts.

13. A cosmetic comprising the fragrance composition according to any one of claims 8 to 12.

14. A cleaning composition comprising the fragrance composition according to any one of claims 8 to 12.

15. A fiber treating agent composition comprising the fragrance composition according to any one of claims 8 to 12.

16. A method of using the fragrance composition according to any one of claims 8 to 11 as a fragrance-imparting component.
